# EUROPEAN PATENT APPLICATION

(11) **EP 1 094 107 A2**
(43) Date of publication of application: **25.04.2001**
(21) Application number: 00122021.9
(22) Date of filing: 10.10.2000
(51) Int. Cl.: C12N 1/20, C12P 13/08

(54) **Fermentative production of L-lysine**

(30) Priority: 22.10.1999 JP 30050099
(71) Applicant: Ajinomoto Co., Inc., Tokyo (JP)
(72) Inventor: Nagase, Kazuo, Fermentation & Biotechnology Labs., Kawasaki-shi, Kanagawa-ken (JP); Yasueda, Hisashi, Fermentation & Biotechnology, Kawasaki-shi, Kanagawa-ken (JP); Sugimoto, Shinichi, Fermentation & Biotechnology, Kawasaki-shi, Kanagawa-ken (JP)
(74) Representative: Strehl Schübel-Hopf & Partner

(57) **Abstract**

A microorganism belonging to the genus Methylobacillus having aspartokinase activity and/or dihydrodipicolinate synthase activity wherein the aspatokinase and the dihydrodipicolinate syntase activity are substantially insensitive to feedback inhibition by L-lysine.

## Description

### FIELD OF THE INVENTION

This invention relates to the microbial industry, especially to a method for the production of L-lysine by fermentation and a microorganism for the production.

### BACKGROUND OF THE ART

In the production of L-Lysine by fermentation, strains isolated from nature or artificial mutants thereof have been used in order to improve the productivity. Many artificial mutants that produce L-lysine are known, and many of them are aminoethylcysteine (AEC) resistant strains and belong to the genus Brevibacterium, Corynebacterium, Bacillus, or Escherichia. Further, various techniques have been disclosed for increasing the amino acid production, for example, use of a transformant obtained by using recombinant DNA (U.S. Patent No. 4,278,765). For example, a method of fermentative production of L-lysine by enhanced expression of wild type dihydrodipicolinate synthase (abbreviated as "DDPS" hereinafter) in Escherichia has been disclosed (U.S. Patent No. 4,346, 170 and Applied Microbiology and Biotechnology, 15, p227-231(1982)). In this case, however, the amplified DDPS is a wild type one and subject to feedback inhibition by L-lysine, thus satisfactory productivity of L-lysine has not been obtained. However, the productivity has been improved drastically by amplifying DDPS and aspartokinase (abbreviated as "AK" hereinafter) insensitive to the feedback inhibition by L-lysine (WO95/16042).

Dihydrodipicolinate synthase is an enzyme that synthesizes dihydrodipicolinate through dehydration condensation of aspartate-beta-semialdehyde and pyruvic acid, and this reaction serves as an entrance into the L-lysine biosynthesis branch in the whole biosynthesis pathway of amino acids derived from aspartic acid. As for microorganism belonging to genus Escherichia, DDPS as well as AK is known as an important rate-limiting enzyme. It is known that DDPS is encoded on the dapA gene in Escherichia coil. The dapA gene has already been cloned and its nucleotide sequence has been determined (Richaud F. et al. J. Bacteriol. 166(1), 297 (1986)).

On the other hand, AK is an enzyme that catalyzes reaction that produces aspartate-beta-semialdehyde from aspartic acid and is an important enzyme that is sensitive to feedback inhibition. E.coli has three isozymes of AK (AKI, AKII and AKIII). Two of them are complex enzymes that also have homoserine dehydrogenase activity (abbreviated as "HD" hereinafter). Only AKIII is an enzyme having a single enzyme activity and is encoded on the lysC gene and also is known to be sensitive to feedback inhibition and repression by L-lysine.

Methods of the fermentative production of an amino acid using methanol, a carbon source that is cheap and easy to obtain in huge amounts, by using several microorganisms, have been known. For example, methods using microorganism belonging to the genus Achromobacter or Pseudomonas (Japanese Patent Publication No. 25273/1970), Protaminobacter (Japanese Patent Application laid-open No. 125590/1974) Protaminobacter or Methanomonas (Japanese Patent Application laid-open No.25790/1975), Micocyclus (Japanese Patent Application laid-open 18886/1977), Methylobacillus (Japanese Patent Application laid-open 91793/1992) and Bacillus (Japanese Patent Application laid-open 505284/1991 , European Patent Application No. 90906690.4) are known.

Especially, as screening methods for microorganisms belonging to the genus Methylobacillus, screening as a mutant resistant to a specific amino acid or metabolic inhibitor of an amino acid (Japanese Patent Application laid-open 91793/1992) and screening as a mutant resistant to halogenated pyruvic acid (Japanese Patent Application laid-open 133788/1994) are known. However, nothing is known whether the method using feedback insensitive DDPS or AK might be effective in the case of the genus Methylobacillus. Especially, nothing is known with respect to a method using recombinant DNA technique and introducing lysine biosynthesis genes into Methylobacillus.

### SUMMARY OF THE INVENTION

The present invention has been accomplished in view of the aforementioned technical problems, and its object is to provide improved method to produce L-lysine by fermentation using methanol as a major carbon source.

The inventors of present invention assiduously studied in order to achieve the forementioned object. As a result, they successfully constructed a Methylobacillus strain producing AK and DDPS that are free from feedback inhibition by L-lysine and have found that the cultivation the constructed strain in a suitable medium results in the accumulation of considerable amounts of L-lysine in the medium. Thus, they accomplished the present invention.

### PREFERRERD EMBODIMENTS OF THE INVENTION

Hereafter, the present invention will be explained in detail.

As the DNA donor, any microorganism that provides DNA that can express AK and/or DDPS activity in Methylobacillus being insensitive to feedback inhibition by L-lysine can be used. It does not matter whether the bacterium is a wild type strain or a mutant strain derived from wild type strain. Preferably, mutants derived from Escherichia coli K-12, mutants derived from Methylobacillus glycogenes NCIMB11375, and mutants derived from Corynebacterium glutamicum, such as Corynebacterium glutamicum ATCC13869, should be used. More preferably, one can obtain feedback-insensitive AK and DDPS gene from Escherichia coli by the method disclosed in WO95/16042. In the case of Corynebacterium glutamicum, it is known that DDPS is not sensitive to feedback inhibition even in the wild type strain (Japanese Patent Application laid-open 62866/1994). It is also suitable as a source of DNA.

Any plasmid vector that contains feedback-insensitive AK and DDPS genes can be used as far as it can be used to transform Methylobacillus strains and can express its character. For example, plasmid pMF42 (gene, 44, 53 (1990)), pRP301, pTB70 (Nature, 287, 396, (1980)) can be used.

Any method of transformation can be used to introduce the plasmid vector into Methylobacillus, as far as it accomplishes good transformation efficiency. For example, a method disclosed in Methods in Enzymology, 118, 640(1986) i.e. the introduction of plasmid DNA into Methylobacillus from E.coli S17-1 strain by conjugation culturing the two strains on the same agar media plate. Direct introduction of plasmid by electroporation can be used (Canadian Journal of Microbiology, 43, 197 (1997)).

As the recipient strain, any Methylobacillus strain can be used. For example, Methylobacillus glycogenes NCIMB11375 or Methylobacillus flagelatum ATCC51484 can be used. By culturing the Methylobacillus strains that has a plasmid comprising AK and DDPS genes in a medium that contains methanol as a major carbon source, considerable amounts of L-lysine can be accumulated in the culture broth.

For introducing the genes encoding feedback-insensitive AK and DDPS, other methods than the above mentioned method using a plasmid can be used, e.g. the introduction of the DNA into the chromosome. Alternatively, a method of modifying feedback-sensitive AK and/or DDPS genes authentically present on the chromosome into feedback-insensitive ones is also effective.

The microorganism used in the present invention can be cultivated by conventional methods for methanol utilizing bacteria. Either nutrient or synthetic medium can be used as far as it contains a carbon source, nitrogen source, inorganic ions and other desired trace organic compounds.

Methanol can be used as a major carbon source, and its content in the medium is from 0.01 to 30% w/v. As the nitrogen source, ammonium sulfate and others can be used in the medium. Furthermore, potassium phosphate, sodium phosphate, manganese sulfate and ferrous sulfate are normally added to the medium.

Cultivation is performed under aerobic condition by shaking or aeration plus agitation, at a pH from 6 to 8, at a temperature from 25 centigrade to 37 centigrade. Cultivation continues from 24 to 120 hours. After the cultivation, the isolation from the broth can be performed by ion-exchange, column chromatography, precipitation or combination of them.

### EXAMPLE

Hereafter, the present invention will be further specifically explained with reference to the following examples.

The compositions of the media used in each example are shown below.

| 〈L medium〉 | |
|---|---|
| Bacto trypton(Difco) | 1% |
| Yeast Extract(Difco) | 0.5% |
| NaCl | 0.5% |

| 〈L agar medium〉 | |
|---|---|
| L medium | |
| Bacto agar (Difco) | 1.5% |

| 〈PY medium〉 | |
|---|---|
| Nutrient Broth (Difco) | 1.0% |
| Yeast extract (Difco) | 0.25% |
| Methanol | 10.0ml/l(pH7.0) |

| 〈PY agar medium〉 | |
|---|---|
| PY medium | |
| Bato agar (Difco) | 1.5% |

| 〈M1 medium〉 | |
|---|---|
| (NH4)2SO4 | 0.2% |
| K2HPO4 | 0.7% |
| KH2PO4 | 0.1% |
| MgSO4 | 0.05% |
| NaCl | 0.01% |
| FeSO4 | 10.0mg/l |
| MnSO4·5H2O | 8.0mg/l |
| Vitamin B1 | 1.0mg/l |
| Biotin | 10.0ug/l |
| Methanol | 5.0ml/l(pH7.0) |

| 〈M1 agar medium〉 | |
|---|---|
| M1 medium | |
| Bacto agar(Difco) | 1.5% |

The pH was adjusted by NaOH or HCl in all cases. The media were steam-sterilized at 120 centigrade for 15min. In the case of media containing methanol, the media except methanol were steam-sterilized and methanol was added afterwards after filtration by Membrane filter 0.45µm (Millipore).

At first, plasmid RSFD80 that is disclosed in WO95/16042 was constructed. The plasmid contains feedback-insensitive DDPS gene, feedback-insensitive AK gene and a replication origin derived from broad host range plasmid. E.coli JM109 which harbors RSFD80 was named AJ12396 and deposited at National Institute of Bioscience and Human-Technology, Agency of Industrial Science and Technology, Ministry of International Trade and Industry (postal code 305-8566, 1-3 Higashi 1-chome, Tsukuba-shi, Ibaraki-ken, Japan) under the accession number FERM BP-4859.

AJ12396 was cultured overnight in 50ml of L medium containing 20mg/l of streptomycin at 37 centigrade and plasmid RSFD80 was purified by Wizard Plus Midipreps DNA Purification System (Promega). In the same manner, pRSFTC was constructed from pVIC40 and pBR322 according to the method disclosed in WO95/16042 (fig.1). JM109 was transformed by pRSFTC and the resultant transformant was cultured overnight in 50ml of L medium containing 10mg/l of tetracyclin at 37 centigrade and pRSFTC was purified by Wizard Plus Midipreps DNA Purification System.

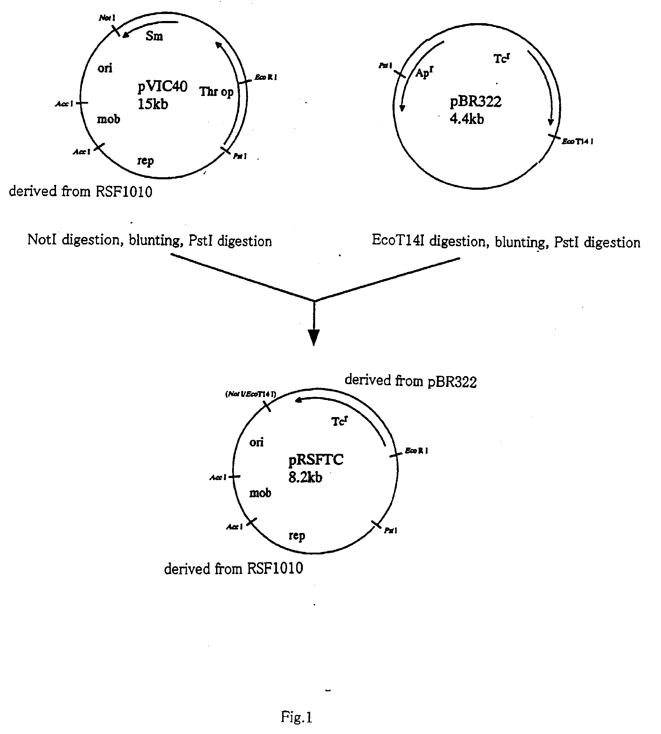

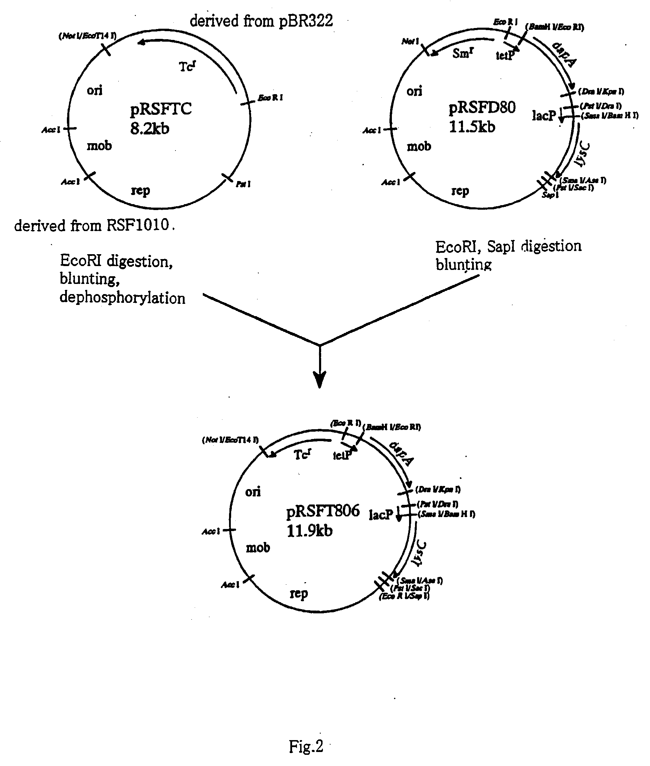

Secondly, RSFD80 was digested by restriction enzyme EcoRI plus SapI. The end was blunted by T4 DNA polymerase. Afterwards, DNA fragments containing AK and DDPS gene were extracted from the agarose gel after electrophoresis using CONCERT Rapid Gel Extraction System (GIBCO BRL). On the other hand, plasmid RSFTC was digested by restriction enzyme EcoRI and blunted by T4 DNA polymerase and dephoshorylated by alkaline phosphatase from E.coli. The dephosphorylated vector was ligated with a DNA fragment that contains AK and DDPS gene and transformed to JM109 (fig.2). Transformants were selected on an agar plate containing 10mg/l of tetracycline. The obtained plasmid was named as pRSFT806. JM109 that contains pRSFT806 and JM109 that contains pRSFTC were used as donor respectively. A tri-parental conjugal plasmid transfer was performed using JM109 that contains PK2013 as a mobilizer and M.glycogenes ATCC29475 was used as an acceptor. The plasmid pRSFT806 and pRSFTC were transferred into M.glycogenes respectively and thus M.glycogenes/pRSFTC and M.glycogenes/pRSF806 were obtained selecting on the M1 agar medium containing 10mg/ml tetracycline.

The obtained M.glycogenes/pRSFTC and M.glycogenes/pRSFT806 were inoculated on M1 medium containing 3% of calcium carbonate and cultivated by shaking at 30 centigrade for 36 hours. After cultivation, the cell bodies were removed by centrifugation and the L-lysine concentration in the supernatant was measured by biotech analyzer AS-210 (Sakura Seiki Co., Ltd.). The results were shown in Table 1.

**Table 1**

| STRAIN | L-LYSINE ACCUMULATION(G/L) |
|---|---|
| M.glycogones/pRSFTC | not detected |
| M.glycogenes/pRSFT806 | 0.1g/l |

## Claims

1. A microorganism belonging to the genus Methylobacillus having aspartokinase activity and/or dihydrodipicolinate synthase activity wherein the aspatokinase and the dihydrodipicolinate syntase activity are substantially insensitive to feedback inhibition by L-lysine.

2. The microorganism according to claim 1, wherein said microorganism is transformed by DNA that encodes aspartokinase and dihydrodipicolinase synthase activity.

3. The microorganism according to claim 2 wherein the DNA is derived from Escherichia coli.

4. The microorganism according to any one of claims 1 to 3, wherein the microorganism is Methylobacillus glycogenes.

5. A method for the production of L-lysine comprising culturing the microorganism according to any one of claims 1 to 4 in the medium that contains methanol as major carbon source and collecting L-lysine accumulated in the culture.
